# EUROPEAN PATENT APPLICATION

(11) **EP 3 336 085 A1**
(43) Date of publication of application: **20.06.2018**
(21) Application number: 16382605.0
(22) Date of filing: 15.12.2016
(51) Int. Cl.: C07D 403/12, C07D 231/12, C07D 231/24, C07D 401/12, C07D 413/12

(54) **PROCESS AND INTERMEDIATE FOR THE PREPARATION OF 1-(4-(2-((1-(3,4-DIFLUOROPHENYL)-1H-PYRAZOL-3-YL)METHOXY)ETHYL)PIPERAZIN-1-YL)ETHANONE**

(71) Applicant: Laboratorios del Dr. Esteve S.A., 08041 Barcelona (ES)
(72) Inventor: Almansa Rosales, Carmen, E-08022 Barcelona (ES); Caamaño Moure, Ana María, E-15705 Santiago de Compostela (ES); Enjo BabÍo, Juan, E-15220 Bertamiráns, Ames (ES)
(74) Representative: ABG Patentes, S.L.

(57) **Abstract**

The present invention relates to a process for the preparation of 1-(4-(2-((1-(3,4-difluorophenyl)-1 H-pyrazol-3-yl)methoxy)ethyl)piperazin-1-yl)ethanone and salts and solvates thereof and to the use of intermediates in the synthesis of these compounds. Also is provided a new intermediate compound.

## Description

### FIELD OF THE INVENTION

The present invention relates to a process for the preparation of 1-(4-(2-((1-(3,4-difluorophenyl)-1H-pyrazol-3-yl)methoxy)ethyl)piperazin-1-yl)ethanone and salts and solvates thereof and to the use of intermediates in the synthesis of these compounds. Also is provided a new intermediate compound.

### BACKGROUND OF THE INVENTION

The compound 1-(4-(2-((1-(3,4-difluorophenyl)-1 H-pyrazol-3-yl)methoxy)ethyl)piperazin-1-yl)ethanone (also named as 1-acetyl-4-(2-{[1-(3,4-difluorophenyl)-1 H-pyrazol-3-yl]methoxy}ethyl)piperazine) is disclosed in WO 2011/147910 (example 39 therein). More recently, PCT/EP2016/070604 describes crystalline salts of this compound with different acids such as hydrochloride, maleate, fumarate, malonate, succinate, oxalate and hydrobromide.

Particularly, its hydrochloride is a highly selective sigma-1 (σ1) receptor antagonist which displays strong analgesic activity, which makes it useful for the treatment and prevention of chronic and acute pain, and particularly, neuropathic pain. It has a molecular weight of 400.85 Da and an optimal physicochemical profile, as indicated by its low basicity (p*K*a of 6.37), its low lipophilicity (log*P* 1.7) and high water solubility (very soluble at both pH=7.4 and pH=2). Its structural formula is as follows:

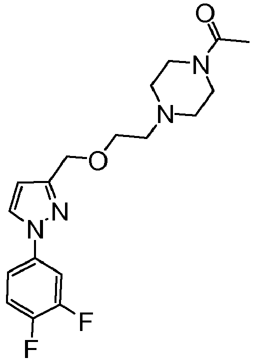

According to the general reaction scheme 1 in WO 2011/147910, compounds described in this application may be prepared through the following route: reaction between hydrazine derivatives (A) and 1,3-diketo derivatives of formula (B) to obtain pyrazole carboxylates of formula (C), reduction to the corresponding alcohols of formula (D) and nucleophilic substitution reaction displacing the leaving group from compounds of formula (E):

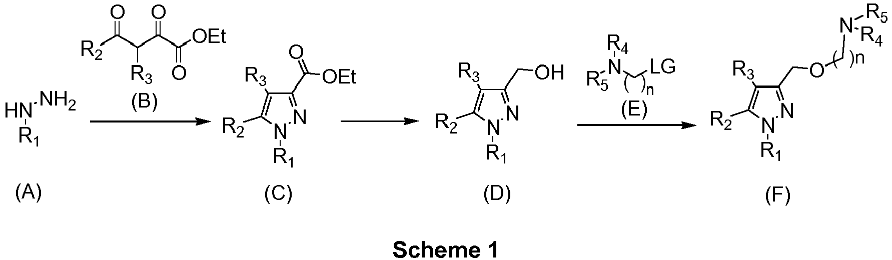

However, when applying the above synthetic route to the compound 1-(4-(2-((1-(3,4-difluorophenyl)-1H-pyrazol-3-yl)methoxy)ethyl)piperazin-1-yl)ethanone the result is not satisfactory for large scale production in industry. In particular, the inventors have found, as it will be explained further hereinafter, that the direct alkylation of the corresponding alcohol of formula (D) (1-(3,4-difluorophenyl)-1 H-pyrazol-3-yl)methanol) with the required compound of formula E (1-(4-(2-chloroethyl)piperazin-1-yl)ethanone) leads to a very low conversion, that was not possible to optimize and affords a very complex crude material which has to be purified by chromatography to obtain the desired product in a low yield (< 30%).

It is therefore desirable to develop a new process for the preparation of 1-(4-(2-((1-(3,4-difluorophenyl)-1H-pyrazol-3-yl)methoxy)ethyl)piperazin-1-yl)ethanone which overcome all or part of the problems associated with the known process belonging to the state of the art, and more suitable for large scale industrial production.

### SUMMARY OF THE INVENTION

The invention faces the problem of providing an improved process for the preparation of 1-(4-(2-((1-(3,4-difluorophenyl)-1H-pyrazol-3-yl)methoxy)ethyl)piperazin-1-yl)ethanone. In particular, the inventors have found that, in contrast to the strategy proposed in the prior art, this compound is more conveniently obtained for purposes of production at large scale through a nucleophilic substitution in which a 1-(3,4-difluorophenyl)-1H-pyrazol-3-yl]methanol derivative acts as an electrophile, being attacked by a suitable nucleophilic reagent that bears the ethylpiperazine moiety. Further, 1-(3,4-difluorophenyl)-1H-pyrazol-3-yl]methanol may be readily obtained through a practical synthetic route.

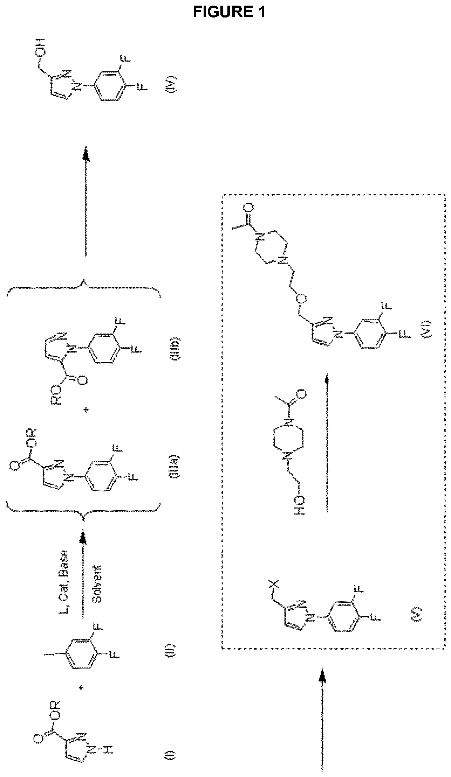

The whole synthetic route is depicted in Figure 1, in which the nucleophilic substitution is highlighted within a dotted square. The process developed by the inventors allows preparing 1-(4-(2-((1-(3,4-difluorophenyl)-1 H-pyrazol-3-yl)methoxy)ethyl)piperazin-1-yl)ethanone (VI) in an efficient manner, without the need of chromatography purification throughout the whole process, using easily available starting materials and applying reaction conditions suitable for large scale production.

One aspect of the present invention relates to a process for preparing 1-(4-(2-((1-(3,4-difluorophenyl)-1 H-pyrazol-3-yl)methoxy)ethyl)piperazin-1-yl)ethanone (VI) or a salt or solvate thereof, said process comprising the reaction between a compound of formula (V) 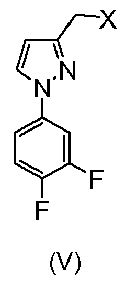 wherein X is a leaving group,
with 1-acetyl-4-(2-hydroxyethyl)piperazine or a salt thereof

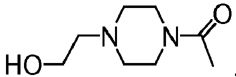

In another aspect, the present invention relates to the use of a compound of general formula (V) as intermediate for obtaining 1-(4-(2-((1-(3,4-difluorophenyl)-1 H-pyrazol-3-yl)methoxy)ethyl)piperazin-1-yl)ethanone (VI) or a salt or solvate thereof.

In another aspect, the present invention relates to the compound of formula (V) wherein the leaving group is tosylate, i.e. [1-(3,4-difluorophenyl)-1 H-pyrazol-3-yl]methyl 4-methylbenzenesulfonate:

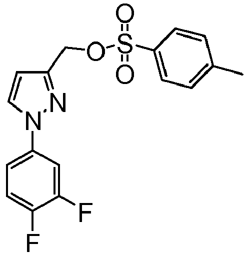

According to a particular embodiment, the compound of formula (V) is obtained from 1-(3,4-difluorophenyl)-1 H-pyrazol-3-yl]methanol (IV)

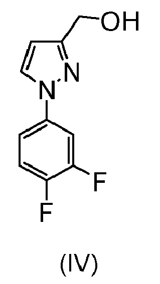

In another aspect, the present invention relates to the use of 1-(3,4-difluorophenyl)-1 H-pyrazol-3-yl]methanol (IV) as intermediate for obtaining 1-(4-(2-((1-(3,4-difluorophenyl)-1 H-pyrazol-3-yl)methoxy)ethyl)piperazin-1-yl)ethanone (VI) or a salt or solvate thereof.

According to a particular embodiment of the present invention, the alcohol of formula (IV) is obtained by reduction of the corresponding pyrazole carboxylate (IIIa) 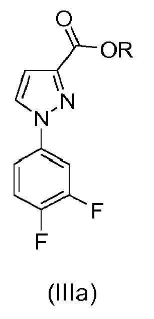 wherein R is a C₁-C₆alkyl.

In another aspect, the present invention relates to the use of the pyrazole carboxylate (IIIa) as intermediate for obtaining 1-(4-(2-((1-(3,4-difluorophenyl)-1H-pyrazol-3-yl)methoxy)ethyl)piperazin-1-yl)ethanone (VI) or a salt or solvate thereof.

According to a particular embodiment, the compound of formula (IIIa) is obtained using Ullmann's reaction conditions by reacting 3,4-difluoroiodobenzene (II) and the corresponding pyrazole carboxylate (I).

These aspects and particulars and preferred embodiments thereof are additionally also defined hereinafter in the detailed description, as well as in the claims.

### BRIEF DESCRIPTION OF DRAWINGS

**Figure 1****.** Scheme with the whole process for preparing 1-(4-(2-((1-(3,4-difluorophenyl)-1 H-pyrazol-3-yl)methoxy)ethyl)piperazin-1-yl)ethanone hydrochloride according to the invention.

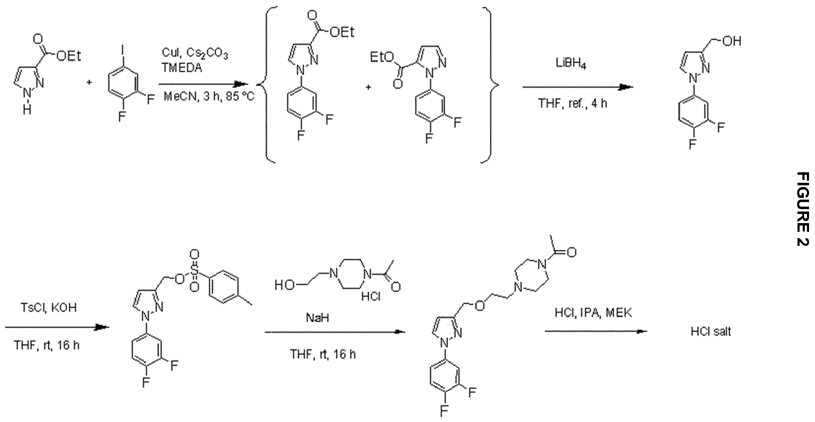

**Figure 2****.** Scheme with the whole process for preparing 1-(4-(2-((1-(3,4-difluorophenyl)-1 H-pyrazol-3-yl)methoxy)ethyl)piperazin-1-yl)ethanone hydrochloride, showing particular reaction conditions, according to an embodiment of the invention.

### DETAILED DESCRIPTION OF THE INVENTION

In the present invention the term "leaving group" refers to a functional group or an atom that can be displaced by another functional group in a substitution reaction, such as a nucleophilic substitution reaction. Suitable leaving groups are well known in the art and may be found in reference books, for example on pages 484-488, of March, J. "Advanced Organic Chemistry: Reactions, Mechanism and Structure", 5th Ed., Wiley-Interscience.. In a particular embodiment, the leaving group is selected from halogen, C₁-C₆ alkylsulfonates, C₆-C₁₀ arylsulfonates and C₁-C₆alkylC₆-C₁₀arylsulfonates, such as chloro, bromo, iodo, mesylate, triflate, tosylate, nosylate and the like.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, camphorsulfonate, etc. Illustrative examples of base addition salts include inorganic base salts such as, for example, ammonium salts and organic base salts such as, for example, ethylenediamine, ethanolamine, *N,N-*dialkylenethanolamine, triethanolamine, glutamine, amino acid basic salts, etc. Illustrative examples of metal salts include, for example, sodium, potassium, calcium, magnesium, aluminum and lithium salts.

Likewise, the compounds described in the present description can be obtained both as free compounds or as solvates (e.g., hydrates, alcoholates, etc.), both forms being included within the scope of the present invention. The solvation methods are generally known in the state of the art. Preferably, the solvate is a hydrate.

The term "organic solvent" includes for example cyclic and acyclic ethers (e.g. Et₂O, iPr₂O, tBu₂O, MeOtBu, 1,4-dioxane, tetrahydrofuran, methyltetrahydrofuran), hydrocarbonated solvents (e.g. pentane, hexane, heptane), halogenated solvents (e.g. dichloromethane, chloroform), aromatic solvents (e.g. toluene, xylene), esters (e.g. EtOAc), nitriles (e.g. acetonitrile), amides (e.g. DMF, DMA), alcohols (e.g. methanol, ethanol, propanol, isopropanol), sulfoxides (DMSO) and mixtures thereof.

By room temperature is meant herein that the reactions or processes are performed without heating or cooling. Generally, by room temperature may be understood as a temperature between about 15 °C and about 30 °C, or more particularly between about 20 °C and about 25 °C.

According to the previously mentioned general reaction scheme 1 in WO 2011/147910, 1-(4-(2-((1-(3,4-difluorophenyl)-1H-pyrazol-3-yl)methoxy)ethyl)piperazin-1-yl)ethanone may be prepared through the following route: reaction between hydrazine derivatives (A) and 1,3-diketo derivatives of formula (B) to obtain pyrazole carboxylates of formula (C), reduction to the corresponding alcohols of formula (D), and nucleophilic substitution reaction displacing the leaving group from compounds of formula (E):

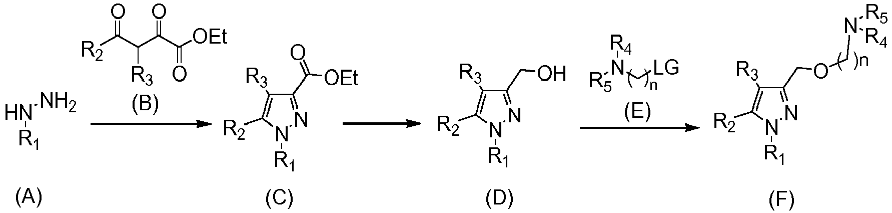

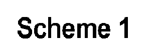

However, all the attempts by the inventors to prepare 1-(4-(2-((1-(3,4-difluorophenyl)-1 H-pyrazol-3-yl)methoxy)ethyl)piperazin-1-yl)ethanone through the above route led to an unsatisfactory result from an industrial point in view. Particularly, under a number of conditions tested, the alkylation that takes place as last step in that route was not able to be completed, even at reflux after some days. In addition to low conversions, it inevitably gave place to a complex mixture containing different by-products that had to be purified by chromatography to give pure 1-(4-(2-((1-(3,4-difluorophenyl)-1 H-pyrazol-3-yl)methoxy)ethyl)piperazin-1-yl)ethanone in a low yield (< 30%).

Initially, the alkylation was carried out with 1-acetyl-4-(2-chloroethyl)piperazine in different reaction conditions and none of them allowed to improve the yield of the reaction over 25%. The use of other alkylation agents instead of 1-acetyl-4-(2-chloroethyl)piperazine (e.g. mesylate, triflate, iodide) was also unsatisfactory.

Moreover, another problem associated with the reaction scheme 1 is that in the first step in the process for the formation of pyrazoles (C), when R₂=H, the reagent B (when R₂=R₃=H) is not commercially available.

Also, hydrazines are recognized as genotoxic compounds (Elder,D.P. et al., J. Pharm. Biomed. Anal., 2011, 54, 900-910; Raman et al., J. Pharm. Biomed. Anal., 2011, 55, 662-667; Derek Nexus Report; Derek Nexus 4.1.0, Nexus 2.0.0) and processes avoiding their use are strongly preferred.

In addition, 3,4-difluorophenylhydrazine is very expensive.

The inventors envisaged alternative approaches for the preparation of 1-(4-(2-((1-(3,4-difluorophenyl)-1 H-pyrazol-3-yl)methoxy)ethyl)piperazin-1-yl)ethanone, and after an extensive research, the inventors have surprisingly found that the synthesis of 1-(4-(2-((1-(3,4-difluorophenyl)-1 H-pyrazol-3-yl)methoxy)ethyl)piperazin-1-yl)ethanone may be successfully accomplished by means of the process object of the present invention.

Thus a new process was designed according to Figure 1, which has the following advantages versus the prior art route shown in scheme 1:
- It avoids the use of genotoxic starting materials.
- It compares favorably in terms of cost: starting 3,4-diflurophenyliodobenzene is substantially cheaper than 3,4-difluorophenylhydrazine.
- It solves the alkylation problem and allows the synthesis of the final compound with much better yield and the elimination of the purification by chromatography, which is not suitable for large scale production.

This new process is characterized by a nucleophilic substitution reaction in which 1-acetyl-4-(2-hydroxyethyl)piperazine or a salt thereof displaces the leaving group of the 1-(3,4-difluorophenyl)-1H-pyrazol-3-yl]methanol derivative of formula (V). Further, a practical synthesis of the compounds of formula (V) is proposed herein. The whole process, as shown in Figure 1, comprises the following steps:
a) Ullmann's reaction between (I) and (II) 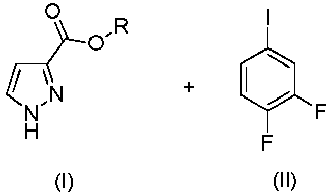 wherein R is a C₁-C₆alkyl,
b) Reduction of the alkyl 3,4-difluorophenylpyrazole carboxylate (IIIa) thus obtained, to the corresponding alcohol (IV),
c) Preparation of the compound of formula (V), and
d) Preparation of 1-(4-(2-((1-(3,4-difluorophenyl)-1 H-pyrazol-3-yl)methoxy)ethyl)piperazin-1-yl)ethanone (VI) or a salt or solvate thereof.

### Conversion of compounds of formula (V) into 1-(4-(2-((1-(3,4-difluorophenyl)-1H-pyrazol-3-yl)methoxy)ethyl)piperazin-1-yl)ethanone (VI) or a salt or solvate thereof

One aspect of the present invention relates to a process for preparing 1-(4-(2-((1-(3,4-difluorophenyl)-1 H-pyrazol-3-yl)methoxy)ethyl)piperazin-1-yl)ethanone (VI) or a salt or solvate thereof, said process comprising a nucleophilic substitution between a compound of formula (V) 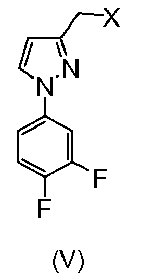 wherein X is a leaving group,
with 1-acetyl-4-(2-hydroxyethyl)piperazine or a salt thereof.

In a particular embodiment, the leaving group is selected from halogen, C₁-C₆ alkylsulfonates, C₆-C₁₀ arylsulfonates and C₁-C₆alkylC₆-C₁₀arylsulfonates, such as chloro, bromo, iodo, mesylate, triflate, tosylate, nosylate and the like. In a preferred embodiment, the leaving group is bromo, mesylate or tosylate, more preferably the leaving group is tosylate.

The reaction may be carried out either with 1-acetyl-4-(2-hydroxyethyl)piperazine or with a salt thereof. In a preferred embodiment, the reaction is carried out with 1-acetyl-4-(2-hydroxyethyl)piperazine hydrochloride, commercially available (Fluorochem ref. 024791) and/or obtainable from the reaction between 2-bromoethanol and 1-acetyl-4-piperazine according to the following reaction scheme 2:

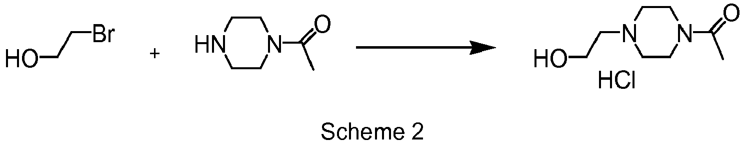

The transformation of compounds of formula (V) into 1-(4-(2-((1-(3,4-difluorophenyl)-1 H-pyrazol-3-yl)methoxy)ethyl)piperazin-1-yl)ethanone (VI) or a salt or solvate thereof is conveniently performed in the presence of a base. Suitable bases include organolithium bases (e.g. nBuLi, tBuLi, sBuLi, MeLi, PhLi, HMDSLi, LDA), alkali metal hydrides (e.g. NaH), alkali metal alcoholates (e.g. NaOMe, NaOEt, NaOiPr, NaOnBu, NaOtBu, KOMe, KOEt, KOiPr, KOnBu, KOtBu), alkali metal hydroxides (e.g. LiOH, NaOH, KOH, CsOH), carbonates (e.g. Cs₂CO₃) and amines (e.g. Et₃N, DIPEA), and mixtures thereof. According to a preferred embodiment, the base is NaOH, KOtBu or NaH, more preferably the base is NaH.

Preferably, the reaction is carried out in the presence of an organic solvent, such as for example a cyclic or acyclic ether (e.g. Et₂O, iPr₂O, tBu₂O, MeOtBu, 1,4-dioxane, tetrahydrofuran, methyltetrahydrofuran), a hydrocarbonated solvent (e.g. pentane, hexane, heptane), a halogenated solvent (e.g. dichloromethane, chloroform), an aromatic solvent (e.g. toluene, xylene), esters (e.g. EtOAc), nitriles (e.g. acetonitrile), amides (e.g. DMF, DMA), alcohols (e.g. methanol, ethanol, propanol, isopropanol), sulfoxides (DMSO) and mixtures thereof. In a particular embodiment, the reaction is performed in the presence of an ether such as THF. In an embodiment, the reaction is carried out at a temperature between -20 °C and 150 °C, preferably between 0 °C and 100 °C and more preferably at room temperature.

According to a preferred embodiment, the reaction is carried out in the presence of NaH in THF at room temperature.

In a particular embodiment, 1-(4-(2-((1-(3,4-difluorophenyl)-1 H-pyrazol-3-yl)methoxy)ethyl)piperazin-1-yl)ethanone (VI) is isolated as an addition salt thereof, e.g. as hydrochloride, maleate, fumarate, malonate, succinate, oxalate or hydrobromide. A preferred salt is hydrochloride, which may successfully crystallize in methyl ethyl ketone using HCI/IPA (isopropyl alcohol).

In another aspect, the present invention relates to the use of a compound of general formula (V), in particular [1-(3,4-difluorophenyl)-1 H-pyrazol-3-yl]methyl 4-methylbenzenesulfonate, as intermediate for obtaining 1-(4-(2-((1-(3,4-difluorophenyl)-1 H-pyrazol-3-yl)methoxy)ethyl)piperazin-1-yl)ethanone (VI) or salt or solvate thereof.

### Preparation of compounds of formula (V)

In another aspect of the invention, the compounds of formula (V), useful for obtaining the compound (VI), may be obtained (scheme 3) from 1-(3,4-difluorophenyl)-1 H-pyrazol-3-yl)methanol (IV).

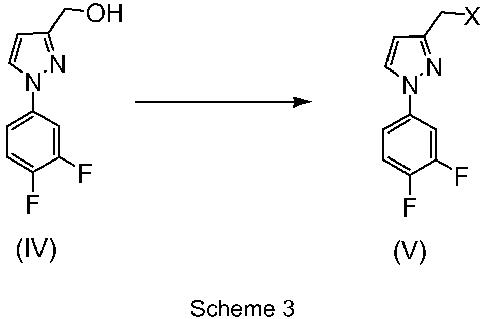

The reaction may be performed in the presence of a base. Suitable bases include organolithium bases (e.g. nBuLi, tBuLi, sBuLi, MeLi, PhLi, HMDSLi, LDA), alkali metal hydrides (e.g. NaH), alkali metal alcoholates (e.g. NaOMe, NaOEt, NaOiPr, NaOnBu, NaOtBu, KOMe, KOEt, KOiPr, KOnBu, KOtBu), alkali metal hydroxides (e.g. LiOH, NaOH, KOH, CsOH), carbonates (e.g. Cs₂CO₃) and amines (e.g. Et₃N, DIPEA) and mixtures thereof. According to a preferred embodiment, the base is NaH, DIPEA or KOH, more preferably the base is KOH.

Preferably, the reaction is carried out in the presence of an organic solvent, such as for example a cyclic or acyclic ether (e.g. Et₂O, iPr₂O, tBu₂O, MeOtBu, tetrahydrofuran (THF), methyltetrahydrofuran (MTHF), 1,4-dioxane), a hydrocarbonated solvent (e.g. pentane, hexane, heptane), a halogenated solvent (e.g. dichloromethane, chloroform), an aromatic solvent (e.g. toluene, xylene), esters (e.g. EtOAc), nitriles (e.g. acetonitrile), amides (e.g. DMF, DMA), alcohols (e.g. methanol, ethanol, propanol, isopropanol), sulfoxides (DMSO) and mixtures thereof. In a particular embodiment, the reaction is performed in the presence of an ether such as THF. In an embodiment, the reaction is carried out at a temperature between -20 °C and 150 °C, preferably between 0 °C and 100 °C and more preferably at room temperature.

In a particular embodiment, the alcohol (IV) was converted into the compound of formula (V) where X is bromo by using CBr₄ and PPh₃ in dichloromethane.

In a particular embodiment, the alcohol (IV) was converted into the compound of formula (V) where X is mesylate by using MsCl and DIPEA.

In a particular embodiment, the alcohol (IV) was converted into the compound of formula (V) where X is tosylate by using TsCl in THF in the presence of a base such as KOH or NaH. In a preferred embodiment the base is KOH.

In a particular embodiment the compound of general formula (V) is [1-(3,4-difluorophenyl)-1 H-pyrazol-3-yl]methyl 4-methylbenzenesulfonate. The present invention is also directed to this compound as such.

In another aspect, the present invention relates to the use of 1-(3,4-difluorophenyl)-1 H-pyrazol-3-yl)methanol (IV) as intermediate for obtaining 1-(4-(2-((1-(3,4-difluorophenyl)-1 H-pyrazol-3-yl)methoxy)ethyl)piperazin-1-yl)ethanone (VI) or a salt or solvate thereof.

### Preparation of 1-(3,4-difluorophenyl)-1H-pyrazol-3-yl)methanol

In another aspect of the invention, 1-(3,4-difluorophenyl)-1 H-pyrazol-3-yl)methanol (IV) may be obtained (scheme 4) by reduction of the corresponding pyrazole carboxylate (IIIa). 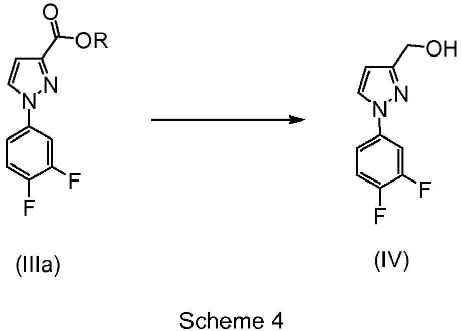 wherein R is a C₁-C₆alkyl. Preferably R is selected from methyl, ethyl, n-propyl, isopropyl, n-butyl, tert-butyl, sec-butyl or pentyl. In a particular embodiment R is ethyl.

The reduction reaction can be carried out under conventional conditions known in the art. Illustrative, non-limiting examples of reducing agents which generate hydride ions and which can be used as reducing agents in the present invention include sodium borohydride (NaBH₄), potassium borohydride (KBH₄), lithium borohydride (LiBH₄), lithium aluminum hydride (LiAlH₄) or lithium triethylborohydride (LiEt₃BH or lithium super-hydride) and mixtures thereof. According to a preferred embodiment, the reducing agent is LiBH₄.

In a particular embodiment, the reduction reaction is carried out using LiBH₄ in THF under reflux.

In a particular embodiment the pyrazole carboxylate (IIIa) is ethyl 1-(3,4-difluorophenyl)-1H-pyrazole-3-carboxylate.

In another aspect, the present invention relates to the use of 1-(3,4-difluorophenyl)-1 H-pyrazole-3-carboxylate (IIIa) as intermediate for obtaining 1-(4-(2-((1-(3,4-difluorophenyl)-1 H-pyrazol-3-yl)methoxy)ethyl)piperazin-1-yl)ethanone (VI) or a salt or solvate thereof.

### Preparation of the required alkyl 1-(3,4-difluorophenyl)-1H-pyrazole-3-carboxylate (IIIa).

In another aspect of the invention, the required pyrazole carboxylate (IIIa) is prepared through the Ullmann's reaction as shown in reaction scheme 5 by reacting 3,4-difluoroiodobenzene and the corresponding pyrazole carboxylate. 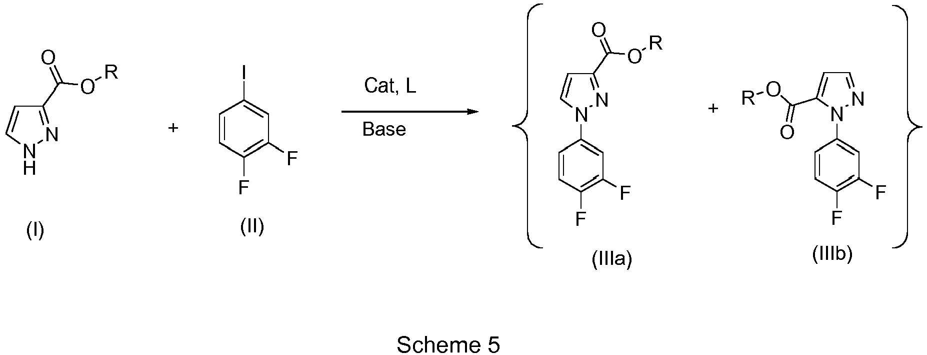 wherein R is a C₁-C₆alkyl. Preferably R is selected from methyl, ethyl, n-propyl, isopropyl, n-butyl, tert-butyl, sec-butyl or pentyl. In a particular embodiment R is ethyl.

Under Ullmann's general reaction temperature and time conditions, using different proportions of catalyst (Cat), ligand (L) and base provided the desired derivative (IIIa). It was surprisingly found that the reaction worked in good yields only using certain conditions, as indicated below for the preferred particular reagents:
Ligands: the ligands (L) are selected from L1, L2, L3, L4, L5, L6, L7, L8, L9 and L10, and mixtures thereof

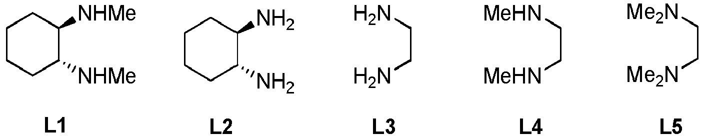

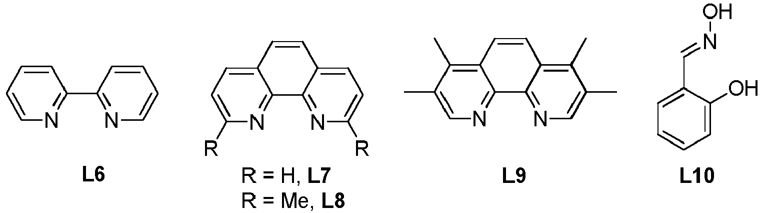

Preferably the ligand (L) is selected from L5 and L10 and more preferably the ligand (L) is L5 (i.e. tetramethylethylenediamine, TMEDA).

Catalyst: the catalyst (Cat) is selected from CuI or Cu₂O, and mixtures thereof. Preferably CuI.

Bases: the bases are selected from Cs₂CO₃, K₃PO₄, K₂CO₃, NaOH, Na₂CO₃, NaHCO₃, and mixtures thereof. Preferably Cs₂CO₃.

Solvents: the solvents are selected from ACN, DMF, THF, Toluene, Dioxane, MTHF, DMA, NMP, MeOH or IPA, and mixtures thereof. Preferably ACN or THF and more preferably ACN.

In a particular embodiment the Ullmann's reaction (scheme 6) is carried out as follows: 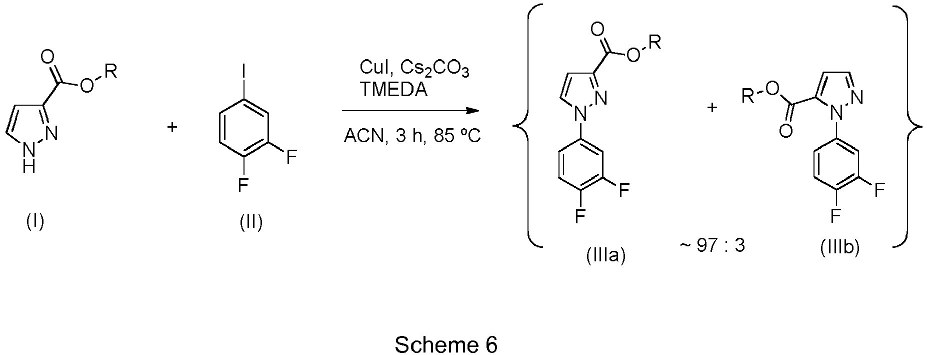 wherein R have the above mentioned meaning.

These particular conditions provided a good proportion of the desired compound IIIa versus undesired IIIb regioisomer which may be removed through a crystallization step.

In a particular embodiment the pyrazole carboxylate (IIIa) is ethyl 1-(3,4-difluorophenyl)-1H-pyrazole-3-carboxylate.

All the features described in this specification, including the claims, description and drawings, can be combined in any way, with the exception of combinations of such mutually exclusive features.

The following examples illustrate the invention and must not be considered in a limiting sense thereof.

### Examples

### 1. Synthesis of ethyl 1-(3,4-difluorophenyl)-1H-pyrazole-3-carboxylate

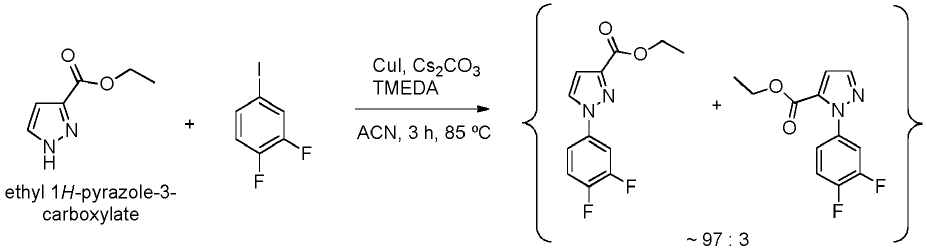

1) Nitrogen atmosphere in the reactor.
2) Load ethyl 1*H*-pyrazole-3-carboxylate.
3) Load CuI (0.2 mol/mol).
4) Load Cs₂CO₃ (1.25 mol/mol).
5) Load acetonitrile (9.5 mL/g) and stirring until homogeneous suspension.
6) Load TMEDA (0.4 mol/mol).
7) Load 3,4-difluoroiodobenzene (1.15 mol/mol).
8) Heat the suspension until reflux and maintain during 5-6 h.
9) Cool until 20-25 °C.
10) Filter the formed salts and washing with acetonitrile (1 mL/g).
11) Load the filtered salts and add water (10 mL/g).
12) Vacuum distil until half of initial volume approximately.
13) Stir the obtained suspension between 30 min. and 60 min. at temperature between 20 °C and 25 °C.
14) Filter the ethyl 1-(3,4-difluorophenyl)-1 H-pyrazole-3-carboxylate obtained as solid.
15) Wash the filtered solid with water (2 x 1 mL/g) and dry it at vacuum.

Yield: 80-85%

### 2. Synthesis of 1-(3,4-difluorophenyl)-1H-pyrazol-3-yl]methanol

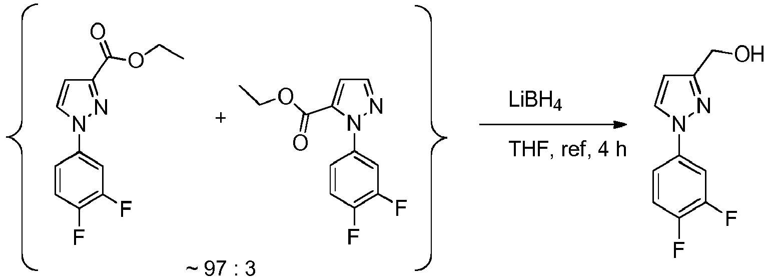

1) Nitrogen atmosphere in the reactor.
2) Load ethyl 3,4-difluorophenyl-1*H*-pyrazole-3-carboxylate.
3) Load THF (4.8 mL/g) and stir until homogeneous suspension.
4) Load LiBH₄ (1.0 mol/mol).
5) Heat the suspension until reflux and maintain during 4-5 h.
6) Cooling until 20-25 °C.
7) Add water (4mL/g) maintaining the temperature between 20 °C and 25 °C.
8) Adjust pH between 4 and 5 and stir from 5 to 15 min.
9) Adjust pH between 6 and 7.
10) Vacuum distil until half of initial volume approximately.
11) Stir the obtained suspension between 30 min. and 60 min. at temperature between 20 °C and 25 °C.
12) Filter the 1-(3,4-difluorophenyl)-1 H-pyrazol-3-yl]methanol obtained as solid.
13) Wash the filtered solid with water (2 x 1 mL/g) and dry it at vacuum.

Yield: 70-95%

### 3. Synthesis of 1-(3,4-difluorophenyl)-1H-pyrazol-3-yl]methyl 4-methylbenzenesulfonate

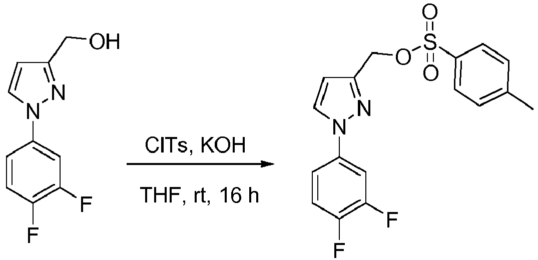

1) Load 1-(3,4-difluorophenyl)-1 H-pyrazol-3-yl]methanol (27.37 g, 130.22 mmol).
2) Load THF (4.75 mL/g; 130 mL)
3) Stir until dissolution.
4) Load KOH (1.33 g/g; 36.53 g, 651.1 mmol).
5) Load a solution of tosyl chloride (1.18 g/g; 32.27 g, 169.29 mmol) in THF (4.75 mL/g; 130 mL).
6) Maintain stirring at room temperature (r.t.) at least 2 h.
7) Load water (9.1 mL/g; 250 mL).
8) Stir until complete dissolution of the present solids.
9) Load EtOAc (11 mL/g; 300 mL) and stir during 5 min.
10) Decanting the phases.
11) Exhaust aqueous phase 1 (AP1) with EtOAc (100 mL).
12) Wash united organic phases with water (100 mL).
13) Dry organic phases (Na₂SO₄) and concentrate until a residue is obtained.
14) Load hexane (50 mL) and concentrate until a residue is obtained.
15)Suspend again the obtained residue in hexane (5.1 mL/g; 140 mL) during 1.5 h at r.t.
16) Filter the solid obtained and wash it with hexane.
17) Dry the [1-(3,4-difluorophenyl)-1 H-pyrazol-3-yl]methyl 4-methylbenzenesulfonate obtained (forced air, 50 °C, 16 h).

Yield: 89%

### 4. Synthesis of 1-acetyl-4-(2-{[1-(3,4-difluorophenyl)-1H-pyrazol-3-yl]methoxy}ethyl)piperazine and its Hydrochloride.

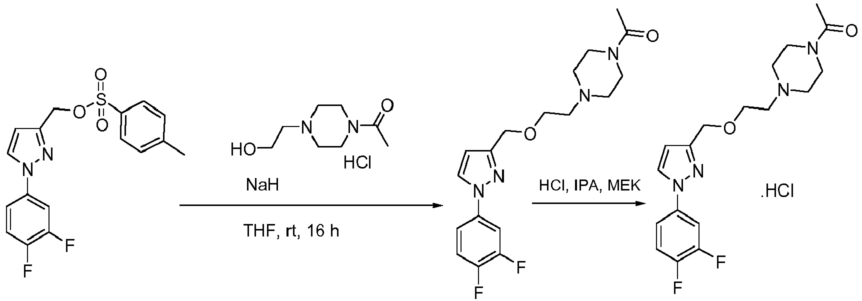

1) Load NaH 60% (0.13 g/g; 2.64 g, 66.00 mmol).
2) Load THF (10.00 mL/g; 200 mL)
3) Stir during 5 min.
4) Load [1-(3,4-difluorophenyl)-1H-pyrazol-3-yl]methyl 4-methylbenzenesulfonate (0.57 g/g; 11.34 g, 65.84 mmol) in portions.
5) Stir during 30 min.
6) Load 1-acetyl-4-(2-hydroxyethyl)piperazine hydrochloride (20.00 g, 54.89 mmol) in portions.
7) Stir at r.t. at least during 3 h.
8) Load water (5 mL/g; 100 mL).
9) Adjust pH between 7 and 7.5 (HCl 37%, 2.0 mL).
10) Remove excess of THF by distillation at reduced pressure.
11)Adjust pH between 2 and 3 (HCl 37%, 5.34 mL).
12) Load EtOAc (3.5 mL/g; 70 mL).
13) Stir during 5 min. and decanting the phases (aqueous phase 1, AP1).
14) Over AP1, load EtOAc (3.5 mL/g; 70 mL).
15) Stir during 5 min. and decanting the phases (aqueous phase 2, AP2).
16) Over AP1, load EtOAc (3.5 mL/g; 70 mL).
17)Adjust pH between 8.5 and 10 (NaOH 20%, 21 mL).
18) Separate phases (aqueous phase 3, AP3 and organic phase 3, OP3).
19) Exhaust AP3 with EtOAc (2 x 70 mL) obtaining OP4 and OP5.
20) Reunite OP3, OP4 and OP5 and concentrate.
21) Load EtOAc (2 mL/g, 40 mL).
22) Filter the insoluble solids.
23) Concentrate and load methyl ethyl ketone (MEK) (2 mL/g, 40 mL).
24) Concentrate and load (MEK) (2 mL/g, 40 mL).
25) Concentrate and load (MEK) (5 mL/g, 100 mL).
26) Heat until 50 °C.
27) Load HCl/IPA 5N (0.55 mL/g; 11 mL, 55.0 mmol).
28) Seed with 1-acetyl-4-(2-{[1-(3,4-difluorophenyl)-1H-pyrazol-3-yl]methoxy}ethyl)piperazine.
29) Slowly decrease the temperature until r.t.
30) Cool between 0 °C and 5 °C and maintain during 1 h.
31) Filter the solid obtained and wash with cool MEK.
32) Dry the 1-acetyl-4-(2-{[1-(3,4-difluorophenyl)-1H-pyrazol-3-yl]methoxy}ethyl)piperazine hydrochloride obtained (100 mbar, 40 °C, 5 h).

Yield: 76%

## Claims

1. A process for preparing 1-(4-(2-((1-(3,4-difluorophenyl)-1 H-pyrazol-3-yl)methoxy)ethyl)piperazin-1-yl)ethanone or a salt or solvate thereof, said process comprising the reaction between a compound of formula (V) 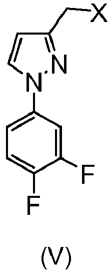 wherein X is a leaving group,
with 1-acetyl-4-(2-hydroxyethyl)piperazine or a salt thereof

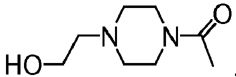

2. The process according to claim 1, wherein the reaction is carried out in the presence of a base.

3. The process according to claim 2, wherein the base is selected from the group consisting of organolithium bases, alkali metal hydrides, alkali metal alcoholates, alkali metal hydroxides, carbonates and amines, or a mixture thereof.

4. The process according to claim 3, wherein the base is selected from the group consisting of nBuLi, tBuLi, sBuLi, MeLi, PhLi, HMDSLi, LDA, NaH, NaOMe, NaOEt, NaOiPr, NaOnBu, NaOtBu, KOMe, KOEt, KOiPr, KOnBu, KOtBu. LiOH, NaOH, KOH, CsOH, Cs₂CO₃, Et₃N and DIPEA, or a mixture thereof.

5. The process according to any one of the preceding claims, wherein the reaction is carried out in the presence of NaH in THF.

6. The process according to any one of the preceding claims, wherein the compound obtained is 1-(4-(2-((1-(3,4-difluorophenyl)-1 H-pyrazol-3-yl)methoxy)ethyl)piperazin-1-yl)ethanone hydrochloride.

7. The process according to any one of the preceding claims, wherein the compound of formula (V) reacted is [1-(3,4-difluorophenyl)-1 H-pyrazol-3-yl]methyl 4-methylbenzenesulfonate.

8. The process according to any one of the preceding claims, said process further comprising obtaining the compound of formula (V) from 1-(3,4-difluorophenyl)-1H-pyrazol-3-yl]methanol.

9. The process according to claim 8, said process further comprising obtaining 1-(3,4-difluorophenyl)-1H-pyrazol-3-yl]methanol from the reduction of a compound of formula (IIIa) 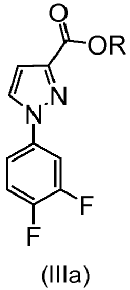 wherein R is a C₁-C₆alkyl.

10. The process according to claim 9 wherein the reduction is performed with a reducing agent selected from the group consisting of NaBH₄, KBH₄, LiBH₄, LiAlH₄ and LiEt₃BH, or a mixture thereof, preferably the reducing agent is LiBH₄.

11. The process according to any one of claims 9 to 10 wherein compound (IIIa) is ethyl 1-(3,4-difluorophenyl)-1 H-pyrazole-3-carboxylate.

12. The process according to any one of claims 9 to 10, said process further comprising obtaining the compound of formula (IIIa) by an Ullmann's reaction between compounds of formula (I) and (II): 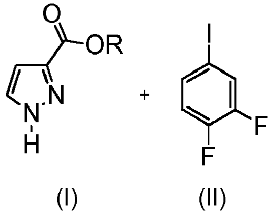 wherein R is C₁-C₆alkyl.

13. The process according to claim 12 wherein the reaction conditions of the Ullmann's reaction are selected from one or more of the following:
the ligand (L) is selected from the group consisting of 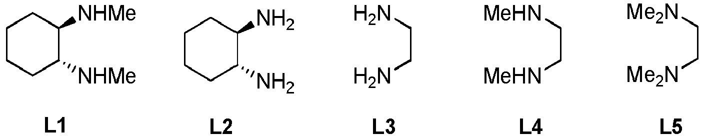 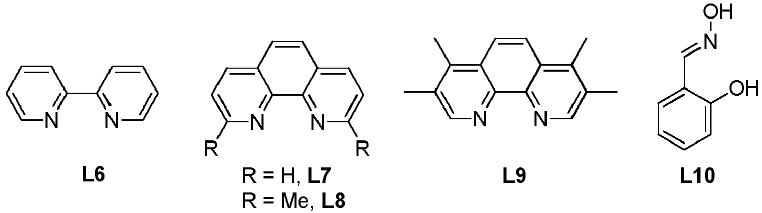 ; and/or
the catalyst (Cat) is selected from the group consisting of CuI and Cu₂O; and/or
the base is selected from the group consisting of Cs₂CO₃, K₃PO₄, K₂CO₃, NaOH, Na₂CO₃, and NaHCO₃; and/or
the solvent is selected from the group consisting of acetonitrile, dimethylformamide, tetrahydrofuran, toluene, dioxane, methyltetrahydrofuran, dimethylacetamide, N-methyl-2-pyrrolidone, methanol and isopropyl alcohol.

14. The process according to claim 13 wherein the ligand is TMEDA (L5), the catalyst is CuI, the base is Cs₂CO₃ and the solvent is acetonitrile.

15. Use of a compound of general formula (V), 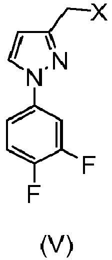 wherein X is a leaving group,
in particular [1-(3,4-difluorophenyl)-1 H-pyrazol-3-yl]methyl 4-methylbenzenesulfonate, for obtaining 1-(4-(2-((1-(3,4-difluorophenyl)-1 H-pyrazol-3-yl)methoxy)ethyl)piperazin-1-yl)ethanone or a salt or solvate thereof.

16. Use of a compound of general formula (IIIa), 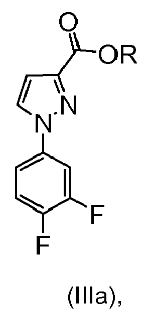 wherein R is C₁-C₆alkyl,
in particular ethyl 1-(3,4-difluorophenyl)-1 H-pyrazole-3-carboxylate, for obtaining 1-(4-(2-((1-(3,4-difluorophenyl)-1 H-pyrazol-3-yl)methoxy)ethyl)piperazin-1-yl)ethanone or a salt or solvate thereof.

17. The compound [1-(3,4-difluorophenyl)-1 H-pyrazol-3-yl]methyl 4-methylbenzenesulfonate
